# EUROPÄISCHE PATENTSCHRIFT

(11) **EP 1 511 517 B1**
(45) Veröffentlichungstag und Bekanntmachung des Hinweises auf die Patenterteilung: **19.09.2007**
(21) Anmeldenummer: 03735445.3
(22) Anmeldetag: 23.05.2003
(51) Int. Cl.: A61K 47/48

(54) **FORMULIERUNG ZUR PARENTERALEN APPLIKATION EINES NA-KANAL-BLOCKERS**
FORMULATION FOR PARENTERAL ADMINISTRATION OF SODIUM CHANNEL BLOCKERS
FORMULATION POUR L'ADMINISTRATION PARENTERALE DE BLOQUEURS DE CANAUX NA

(30) Priorität: 29.05.2002 DE 10223783
(43) Veröffentlichungstag der Anmeldung: 09.03.2005
(73) Patentinhaber: Boehringer Ingelheim Pharma GmbH & Co. KG, 55216 Ingelheim am Rhein (DE)
(72) Erfinder: KRUSS, Bernd, 88454 Hochdorf (DE); MAUZ, Annerose, 88515 Langenenslingen-Emerfeld (DE); RÜHR, Karin, 88400 Biberach (DE); STASSEN, Jean-Marie, B-3210 Lubbeek (BE); VEIT, Claus, 884000 Biberach (DE); WAGNER, Klaus, 88447 Warthausen (DE)
(86) Internationale Anmeldenummer: PCT/EP2003/005399
(87) Internationale Veröffentlichungsnummer: WO 2003/099336

(56) Entgegenhaltungen:
- WO-A-95/17191
- WO-A-99/14199
- TAYLOR C P ET AL: "Nachannels as targets for neuroprotective drugs" TRENDS IN PHARMACOLOGICAL SCIENCES, ELSEVIER TRENDS JOURNAL, CAMBRIDGE, GB, Bd. 16, Nr. 9, September 1995 (1995-09), Seiten 309-316, XP004207535 ISSN: 0165-6147
- T. ANGER ET AL.: "Medicinal chemistry of neuronal voltage-gated sodium channel blockers" JOURNAL OF MEDICINAL CHEMISTRY, Bd. 44, Nr. 2, 2001, Seiten 115-137, XP001164134 USA

## Beschreibung

Die Erfindung betrifft eine neue Formulierung von (-)-(1*R*,2"*S*)-2-(2"-Benzyloxy)-propyl-4'-hydroxy-5,9,9-trimethyl-6,7-benzomorphan (BIII 890) oder eines seiner pharmakologisch verträglichen Salze, insbesondere seines Hydrochlorids, enthaltend einen Komplex aus dem Wirkstoff und einem Cyclodextrin, insbesondere Hydroxypropyl-γ-Cyclodextrin, gegebenenfalls in Gegenwart einer Hydroxysäure, zur parenteralen, insbesondere zur intravenösen Anwendung, deren Herstellung und Verwendung.

Mit den Bezeichnungen "BIII 890" und "Wirkstoff" ist stets die aus der WO 99/14199 bekannte Verbindung (-)-(1 *R*,2"*S*)-2-(2"-Benzyloxy)propyl-4'-hydroxy-5,9,9-trimethyl-6,7-benzomorphan der Formel

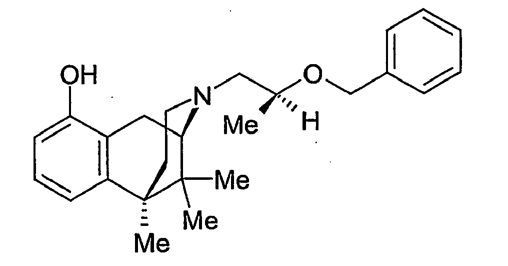

in Form der freien Base oder der entsprechenden Säureadditionssalze mit pharmakologisch unbedenklichen Säuren, insbesondere in Form ihres Hydrochlorids, gemeint. Andere Bezeichnungen für BIII 890 sind Crobenetine und [2*R*-[2,3(*S**),6]]-1,2,3,4,5,6-Hexahydro-6,11,11-trimethyl-3-[2-(phenylmethoxy)propyl]-2,6-methano-3-benzazocin-10-ol. BIII 890 ist ein Natrium-Kanal-Blocker mit neuroprotektiven Eigenschaften; die Hauptindikationsgebiete sind thromboembolischer Schlaganfall, Hirnverletzungen und Schmerz.

Aufgabe der Erfindung ist es, eine neue Formulierung für den Wirkstoff BIII 890, insbesondere für dessen Hydrochlorid, bereitzustellen.

Gegenstand der Erfindung sind pharmazeutische Zusammensetzungen umfassend den Wirkstoff BIII 890 oder eines seiner pharmazeutisch verträglichen Salze, insbesondere sein Hydrochlorid, und ein Cyclodextrin-Derivat, insbesondere gamma-Cyclodextrin (γ-CD), Hydroxypropyl-gamma-cyclodextrin (HP-γ-CD), Hydroxypropylbeta-cyclodextrin (HP-β-CD) oder Sulfobutylether-beta-cyclodextrin (SBE-β-CD). Das bevorzugte Cyclodextrin-Derivat ist Hydroxypropyl-γ-cyclodextrin. Hydroxypropyl-γ-cyclodextrin mit einem molaren Substitutionsgrad von 0,5 bis 0,7 ist beispielsweise von der Firma Wacker-Chemie GmbH, D-Burghausen, unter dem Namen "CAVASOL ® W8 HP Pharma" im Handel. "CAVASOL ® W8 HP Pharma" ist für die erfindungsgemäße pharmazeutische Zusammensetzung besonders bevorzugt. Die Verwendung von bestimmten Cyclodextrinen zur Löslichkeitsverbesserung etwa von Carbamazepin wird in der internationalen Anmeldung WO 95/17191 beschrieben. Gemäß Trends in Pharmacological Sciences, Elsevier Trends Journal, Cambridge, GB (09-1995), 16(9), 306-316 ist Carbamazepin ein Natriumkanalblocker mit neuroprotektiven Eigenschaften.

Für die oben genannten Indikationen ist eine leichte Steuerbarkeit der Dosierung erforderlich, um die Aufrechterhaltung von steady-state Plasmaspiegeln sicher gewährleisten zu können. BIII 890 wird daher zweckmäßigerweise parenteral verabreicht.

Neben dem Wirkstoff und dem Cyclodextrin-Derivat können die zur parenteralen Anwendung bestimmten erfindungsgemäßen pharmazeutischen Zusammensetzungen Hydroxysäuren wie beispielsweise Äpfelsäure, Milchsäure, Weinsäure oder Zitronensäure enthalten. Gegebenenfalls enthalten sie ferner übliche Hilfs- und Trägerstoffe wie beispielsweise die Isotonanzien Glucose, Mannitol oder Natriumchlorid oder Natriumacetat bzw. Natriumacetat-Trihydrat als Puffer in Verbindung mit Essigsäure oder einen Zitronensäure/Phosphat-Puffer bestehend z.B. aus Zitronensäure und Dinatriumhydrogenphosphat bzw. Dinatriumhydrogenphosphat-Dihydrat. Als Lösungsmittel dient üblicherweise Wasser für Injektionszwecke.

Im folgenden sollen die einzelnen Ausführungsformen der Erfindung näher erläutert werden.

### Komplexierung des Wirkstoffs mit Cyclodextrinen

Zu den geeigneten Cyclodextrinen gehören beispielsweise substituierte β-Cyclodextrin (bestehend aus 7 Glucopyranoseeinheiten), Hydroxypropyl-β-Cyclodextrin (HPβCD), Sulfobutylether-β-Cyclodextrin (SBEßCD), γ-Cyclodextrin (bestehend aus 8 Glucopyranoseeinheiten) und Hydroxypropyl-γ-Cyclodextrin (HPγCD).

Löslichkeitsexperimente bzw. ¹H-NMR-Spektren zeigen überraschenderweise, daß γ-Cyclodextrin sowie Hydroxypropyl-γ-Cyclodextrin (HPγCD), Hydroxypropyl-β-Cyclodextrin (HPβCD) und Sulfobutylether-β**-**cyclodextrin (SBEβCD) das Hydrochlorid von BIII 890 komplexieren.

### Komplexierung des Wirkstoffs mit Cyclodextrinen und Hydroxysäuren

Eine weitere Ausführungsform der Erfindung betrifft die Komplexierung des Wirkstoffs oder eines seiner Salze mit Cyclodextrinen und Hydroxysäuren. Dabei kann die benötigte Menge an Cyclodextrin durch die Bildung eines ternären Komplexes bestehend aus BIII 890, dem jeweiligen Cyclodextrin und einer Hydroxysäure reduziert werden. Zu den geeigneten Cyclodextrinen gehören beispielsweise, Hydroxypropyl-β-Cyclodextrin (HPβCD), Sulfobutylether-β-cyclodextrin (SBEßCD), γ-Cyclodextrin und Hydroxypropyl-γ-Cyclodextrin (HPγCD). Als Hydroxysäuren kommen beispielsweise Äpfelsäure, Milchsäure, Weinsäure und Zitronensäure in Frage.

Eine erfindungsgemäße Ausführung einer parenteralen Zubereitung von BIII 890 oder eines seiner physiologisch verträglichen Salze, wie z.B. das Hydrochlorid, enthält den Wirkstoff in Dosierungen von 1 mg/kg Körpergewicht bis 30 mg/kg Körpergewicht täglich, vorzugsweise im Bereich 3-15 mg/kg Körpergewicht. Die Mengen-, Konzentrations- bzw. Dosierungsangaben beziehen sich stets auf die Wirkstoffbase, unabhängig davon, ob BIII 890 in Form der "Base" (= Verbindung der auf Seite 1 angegebenen Formel) oder in Form eines der pharmakologisch verträglichen Salze eingesetzt wird.

Die Anwendung erfolgt vorzugsweise über eine kontinuierliche Infusion über 24 Stunden oder gegebenenfalls über mehrere Tage, um einen Steady-State Plasmaspiegel aufrechtzuerhalten. Die Applikationsvolumina liegen im Bereich von 50 bis 500 ml, vorzugsweise bei 100 bis 250 ml, d.h. die Anwendungskonzentrationen des Wirkstoffs liegen im Bereich 150 mg/500 ml = 0,3 mg/ml (0,03 %) bis 1500 mg/100 ml = 15 mg/ml (1,5 %). Bevorzugt ist eine Wirkstoffkonzentration von 0,5 mg/ml = 0,05 % (g/v) bis 3,5 mg/ml = 0,35 % (g/v).

Das molare Verhältnis von Wirkstoff zu Cyclodextrin liegt erfindungsgemäß zwischen 1:1 und 1:5. Bevorzugt ist ein molares Verhältnis von 1 : 2,5 bis 1 : 3,5. In Gegenwart von Hydroxysäure liegt das molare Verhältnis von Wirkstoff zu Cyclodextrin erfindungsgemäß zwischen 1 : 0,5 und 1 : 3; bevorzugt ist ein molares Verhältnis von 1:0,5 bis 1:1,5.

Die folgenden Beispiele sollen die Erfindung näher erläutern:

### BEISPIELE

### Beispiel 1:

lnfusions-/Injektionslösung enthaltend einen Hydroxypropyl-γ-Cyclodextrin-Komplex

| | |
|---|---|
| BIII 890 Hydrochlorid | 767 mg* |
| **HpγCD**)** | **10000 mg** |
| Mannitol | 11000 mg |
| Essigsäure 99% | 125,25 mg |
| Natriumacetat-Trihydrat | 56,5 mg |
| Wasser für Injektionszwecke ad | 250 ml |

| | |
|---|---|
| *) entspricht 700 mg BIII 890 in Form der Base **) beispielsweise "CAVASOL ® W8 HP Pharma" von Wacker | |

### Beispiel 2:

Infusions-/Injektionslösung enthaltend einen γ-Cyclodextrin-Komplex

| | |
|---|---|
| BIII 890 CL | 383,5 mg*** |
| **γCD** | **5000 mg** |
| NaCl | 2250 mg |
| Wasser für Injektionszwecke ad | 250 ml |

| | |
|---|---|
| ***) entspricht 350 mg BIII 890 in Form der Base | |

### Beispiel 3:

Infusions-/Injektionslösung enthaltend einen Hydroxypropyl-β-Cyclodextrin-Komplex

| | |
|---|---|
| BIII 890 CL | 767 mg* |
| **HPβCD** | **7500mg** |
| Mannitol | 12500 mg |
| Essigsäure 99% | 125,25 mg |
| Natriumacetat-Trihydrat | 56,5 mg |
| Wasser für Injektionszwecke ad | 250 ml |

| | |
|---|---|
| *) entspricht 700 mg BIII 890 in Form der Base | |

### Beispiel 4:

Infusions-/Injektionslösung enthaltend einen Sulfobutylether-β-Cyclodextrin-Komplex

| | |
|---|---|
| BIII 890 CL | 767 mg* |
| **SBEβCD** | **5000 mg** |
| Mannitol | 12500 mg |
| Essigsäure 99% | 125,25 mg |
| Natriumacetat-Trihydrat | 56,5 mg |
| Wasser für Injektionszwecke ad | 250 ml |

| | |
|---|---|
| *) entspricht 700 mg BIII 890 in Form der Base | |

### Beispiel 5:

Infusions-/Injektionslösung enthaltend einen Hydroxypropyl-γ-Cyclodextrin-Komplex in Gegenwart von Hydroxysäuren

| | |
|---|---|
| BIII 890 CL | 767 mg* |
| **HPγCD** | **2500 mg** |
| Citronensäure | 708 mg |
| Mannitol | 12500 mg |
| Essigsäure 99% | 125,25 mg |
| Natriumacetat-Trihydrat | 56,5 mg |
| Wasser für Injektionszwecke ad | 250 ml |

| | |
|---|---|
| *) entspricht 700 mg BIII 890 in Form der Base | |

### Beispiel 6:

Infusions-/Injektionslösung enthaltend einen γ-Cyclodextrin-Komplex in Gegenwart einer Hydroxysäure

| | |
|---|---|
| BIII 890 CL | 767 mg* |
| **γCD** | **2500 mg** |
| Weinsäure | 138,25 mg |
| NaCl | 2250 mg |
| Wasser für Injektionszwecke ad | 250 ml |

| | |
|---|---|
| *) entspricht 700 mg BIII 890 in Form der Base | |

Durch Gabe eines bestimmten Volumens einer der oben beschriebenen Lösungen kann die verabreichte Menge des Wirkstoffs gesteuert werden. Beispielsweise entspricht die tägliche Applikation von 100 ml einer Lösung gemäß Beispiel 1 einer Gabe von 280 mg BIII 890 täglich.

## Patentansprüche

1. Pharmazeutische Zusammensetzung zur parenteralen Anwendung enthaltend einen Cyclodextrin-Komplex von BIII 890 oder eines seiner pharmakologisch verträglichen Salze.

2. Pharmazeutische Zusammensetzung gemäß Anspruch 1, wobei es sich bei dem Cyclodextrin-Komplex um einen ternären Komplex bestehend aus BIII 890 oder eines seiner pharmakologisch verträglichen Salze, einem Cyclodextrin und einer Hydroxysäure handelt.

3. Pharmazeutische Zusammensetzung gemäß Anspruch 1 oder 2, **dadurch gekennzeichnet, dass** der Cyclodextrin-Komplex von einem substituierten oder unsubstituierten Cyclodextrin gebildet wird.

4. Pharmazeutische Zusammensetzung gemäß einem der Ansprüche 1 bis 3, wobei das Cyclodextrin aus der Gruppe bestehend aus γ-CD, HP-γ-CD, HP-β-CD und SBE-β-CD ausgewählt ist.

5. Pharmazeutische Zusammensetzung gemäß einem der Ansprüche 1 bis 4, wobei als Cyclodextrin HP-γ-CD verwendet wird.

6. Pharmazeutische Zusammensetzung gemäß einem der Ansprüche 2 bis 5, wobei die Hydroxysäure aus der Gruppe bestehend aus Äpfelsäure, Milchsäure, Weinsäure und Zitronensäure ausgewählt ist.

7. Pharmazeutische Zusammensetzung gemäß einem der Ansprüche 2 bis 6, wobei als Hydroxysäure Zitronensäure verwendet wird.

8. Pharmazeutische Zusammensetzung nach einem der Ansprüche 1 bis 7, bei der das Hydrochlorid von BIII 890 als Wirkstoff verwendet wird.

9. Pharmazeutische Zusammensetzung gemäß Anspruch 1 oder 3 enthaltend das Hydrochlorid von BIII 890 und HP-γ-CD.

10. Pharmazeutische Zusammensetzung gemäß Anspruch 2 enthaltend das Hydrochlorid von BIII 890, HP-γ-CD und Zitronensäure.

11. Pharmazeutische Zusammensetzung gemäß einem der voranstehenden Ansprüche, **dadurch gekennzeichnet, daß** sie zur intravenösen Anwendung bestimmt ist.

12. Pharmazeutische Zusammensetzung gemäß einem der voranstehenden Ansprüche, **dadurch gekennzeichnet, daß** die Wirkstoff-Konzentration zwischen 0,3 mg/ml und 15 mg/ml liegt.

13. Pharmazeutische Zusammensetzung gemäß einem der voranstehenden Ansprüche, **dadurch gekennzeichnet, daß** die Wirkstoff-Konzentration zwischen 0,5 mg/ml und 3,5 mg/ml liegt.

14. Pharmazeutische Zusammensetzung gemäß einem der voranstehenden Ansprüche, **dadurch gekennzeichnet, daß** das molare Wirkstoff-Cyclodextrin-Verhältnis zwischen 1:1 und 1:5 liegt.

15. Verwendung
a) eines Cyclodextrin-Komplexes oder
b) eines Cyclodextrin-Komplexes in Gegenwart einer Hydroxysäure
zur Herstellung eines Arzneimittels zur parenteralen Gabe enthaltend BIII 890 oder eines seiner pharmakologisch verträglichen Salze zur Behandlung von Schlaganfall.

16. Verwendung gemäß Anspruch 15, wobei das Cyclodextrin eine substituiertes γ-Cyclodextrins ist.

17. Verwendung gemäß Anspruch 15 oder 16, wobei BIII 890 in Form eines seiner pharmakologisch verträglichen Säureadditionssalze, insbesondere als Hydrochlorid, verwendet wird.

18. Verwendung gemäß einem der Ansprüche 15 bis 17, wobei die Hydroxysäure Äpfelsäure, Milchsäure, Weinsäure oder Zitronensäure ist.

19. Verwendung gemäß Anspruch 18, wobei die Hydroxysäure Weinsäure oder Äpfelsäure ist.

20. Verwendung gemäß einem der Ansprüche 15 bis 19, **dadurch gekennzeichnet, daß** die pharmazeutische Zusammensetzung zur intravenösen Anwendung bestimmt ist.

21. Verwendung gemäß einem der Ansprüche 15 bis 20, **dadurch gekennzeichnet, daß** die Wirkstoff-Konzentration zwischen 0,3 mg/ml und 15 mg/ml liegt.

22. Verwendung gemäß einem der Ansprüche 15 bis 20, **dadurch gekennzeichnet, daß** die Wirkstoff-Konzentration zwischen 0,5 mg/ml und 3,5 mg/ml liegt.

23. Verwendung gemäß einem der Ansprüche 15 bis 20, **dadurch gekennzeichnet, daß** das molare Wirkstoff-Cyclodextrin-Verhältnis zwischen 1:1 und 1:5 liegt.

24. Verwendung gemäß einem der Ansprüche 15 bis 20 zur Behandlung von akutem Schlaganfall.

## Claims

1. Pharmaceutical composition for parenteral use containing a cyclodextrin complex of BIII 890 or one of the pharmacologically acceptable salts thereof.

2. Pharmaceutical composition according to claim 1, wherein the cyclodextrin complex is a ternary complex consisting of BIII 890 or one of the pharmacologically acceptable salts thereof, a cyclodextrin and a hydroxy acid.

3. Pharmaceutical composition according to claim 1 or 2, **characterised in that** the cyclodextrin complex is formed by a substituted or unsubstituted cyclodextrin.

4. Pharmaceutical composition according to one of claims 1 to 3, wherein the cyclodextrin is selected from the group consisting of γ-CD, HP-γ-CD, HP-β-CD and SBE-β-CD.

5. Pharmaceutical composition according to one of claims 1 to 4, wherein HP-γ-CD is used as the cyclodextrin.

6. Pharmaceutical composition according to one of claims 2 to 5, wherein the hydroxy acid is selected from the group consisting of malic acid, lactic acid, tartaric acid and citric acid.

7. Pharmaceutical composition according to one of claims 2 to 6, wherein citric acid is used as the hydroxy acid.

8. Pharmaceutical composition according to one of claims 1 to 7, wherein the hydrochloride of BIII 890 is used as the active substance.

9. Pharmaceutical composition according to claim 1 or 3 containing the hydrochloride of BIII 890 and HP-γ-CD.

10. Pharmaceutical composition according to claim 2 containing the hydrochloride of BIII 890, HP-γ-CD and citric acid.

11. Pharmaceutical composition according to one of the preceding claims, **characterised in that** it is intended for intravenous administration.

12. Pharmaceutical composition according to one of the preceding claims, **characterised in that** the concentration of active substance is between 0.3 mg/ml and 15 mg/ml.

13. Pharmaceutical composition according to one of the preceding claims, **characterised in that** the concentration of active substance is between 0.5 mg/ml and 3.5 mg/ml.

14. Pharmaceutical composition according to one of the preceding claims, **characterised in that** the molar ratio of active substance to cyclodextrin is between 1:1 and 1:5.

15. Use of
a) a cyclodextrin complex or
b) a cyclodextrin complex in the presence of a hydroxy acid
for preparing a pharmaceutical composition for parenteral administration containing BIII 890 or one of the pharmacologically acceptable salts thereof for the treatment of stroke.

16. Use according to claim 15, wherein the cyclodextrin is a substituted γ-cyclodextrin.

17. Use according to claim 15 or 16, wherein BIII 890 is used in the form of one of the pharmacologically acceptable acid addition salts thereof, particularly the hydrochloride thereof.

18. Use according to one of claims 15 to 17, wherein the hydroxy acid is malic acid, lactic acid, tartaric acid or citric acid.

19. Use according to claim 18, wherein the hydroxy acid is tartaric acid or malic acid.

20. Use according to one of claims 15 to 19, **characterised in that** the pharmaceutical composition is intended for intravenous administration.

21. Use according to one of claims 15 to 20, **characterised in that** the concentration of active substance is between 0.3 mg/ml and 15 mg/ml.

22. Use according to one of claims 15 to 20, **characterised in that** the concentration of active substance is between 0.5 mg/ml and 3.5 mg/ml.

23. Use according to one of claims 15 to 20, **characterised in that** the molar ratio of active substance to cyclodextrin is between 1:1 and 1:5.

24. Use according to one of claims 15 to 20 for the treatment of acute stroke.

## Revendications

1. Composition pharmaceutique pour l'application parentérale contenant un complexe de cyclodextrine de BIII 890 ou de l'un de ses sels pharmacologiquement acceptables.

2. Composition pharmaceutique selon la revendication 1 où le complexe de cyclodextrine est un complexe ternaire consistant en BIII 890 ou l'un de ses sels pharmacologiquement acceptables, une cyclodextrine et un hydroxyacide.

3. Composition pharmaceutique selon la revendication 1 ou 2 **caractérisée en ce que** le complexe de cyclodextrine est formé par une cyclodextrine substituée ou non substituée.

4. Composition pharmaceutique selon l'une des revendications 1 à 3 où la cyclodextrine est choisie dans le groupe consistant en γ-CD, HP-γ-CD, HP-β-CD et SBE-β-CD.

5. Composition pharmaceutique selon l'une des revendications 1 à 4 où HP-γ-CD est utilisée comme cyclodextrine.

6. Composition pharmaceutique selon l'une des revendications 2 à 5 où l'hydroxyacide est choisi dans le groupe consistant en l'acide malique, l'acide lactique, l'acide tartrique et l'acide citrique.

7. Composition pharmaceutique selon l'une des revendications 2 à 6 où l'acide citrique est utilisé comme hydroxyacide.

8. Composition pharmaceutique selon l'une des revendications 2 à 7 où le chlorhydrate de BIII 890 est utilisé comme principe actif.

9. Composition pharmaceutique selon la revendication 1 ou 3 contenant le chlorhydrate de BIII 890 et HP-γ-CD.

10. Composition pharmaceutique selon la revendication 2 contenant le chlorhydrate de BIII 890, HP-γ-CD et de l'acide citrique.

11. Composition pharmaceutique selon l'une des revendications précédentes **caractérisée en ce qu'**elle est conçue pour l'utilisation intraveineuse.

12. Composition pharmaceutique selon l'une des revendications précédentes **caractérisée en ce que** la concentration de principe actif est située entre 0,3 mg/ml et 15 mg/ml.

13. Composition pharmaceutique selon l'une des revendications précédentes **caractérisée en ce que** la concentration de principe actif est située entre 0,5 mg/ml et 3,5 mg/ml.

14. Composition pharmaceutique selon l'une des revendications précédentes **caractérisée en ce que** le rapport molaire principe actif-cyclodextrine est situé entre 1 : 1 et 1 : 5.

15. Utilisation
a) d'un complexe de cyclodextrine ou
b) d'un complexe de cyclodextrine en présence d'un hydroxyacide
pour la production d'un médicament pour l'administration parentérale contenant BIII 890 ou l'un de ses sels pharmacologiquement acceptables pour le traitement de l'apoplexie cérébrale.

16. Utilisation selon la revendication 15 où la cyclodextrine est une γ-cyclodextrine substituée.

17. Utilisation selon la revendication 15 ou 16 où BIII 890 est utilisé sous forme de l'un de ses sels d'addition d'acide pharmacologiquement acceptables, en particulier sous forme de chlorhydrate.

18. Utilisation selon l'une des revendications 15 à 17 où l'hydroxyacide est l'acide malique, l'acide lactique, l'acide tartrique ou l'acide citrique.

19. Utilisation selon la revendication 18 où l'hydroxyacide est l'acide tartrique ou l'acide malique.

20. Utilisation selon l'une des revendications 15 à 19 **caractérisée en ce que** la composition pharmaceutique est conçue pour l'application intraveineuse.

21. Utilisation selon l'une des revendications 15 à 20 **caractérisée en ce que** la concentration de principe actif est située entre 0,3 mg/ml et 15 mg/ml.

22. Utilisation selon l'une des revendications 15 à 20 **caractérisée en ce que** la concentration de principe actif est située entre 0,5 mg/ml et 3,5 mg/ml.

23. Utilisation selon l'une des revendications 15 à 20 **caractérisée en ce que** le rapport molaire principe actif-cyclodextrine est situé entre 1 : 1 et 1 : 5.

24. Utilisation selon l'une des revendications 15 à 20 pour le traitement de l'apoplexie cérébrale aiguë.
